# FASCICULE DE BREVET EUROPEEN

(11) **EP 1 495 325 B1**
(45) Date de publication et mention de la délivrance du brevet: **13.05.2009**
(21) Numéro de dépôt: 03725266.5
(22) Date de dépôt: 05.03.2003
(51) Int. Cl.: G01N 33/531, C07K 16/44, C07H 19/02, G01N 33/53

(54) **UTILISATION DE DERIVES DE NUCLEOSIDES COMPORTANT UN GROUPEMENT CITRATE POUR LA PRODUCTION D'ANTICORPS AYANT UNE AFFINITE POUR DES NUCLEOSIDES TRI-PHOSPHORYLES**
VERWENDUNG DES DERIVATEN VON NUCLEOSIDEN MIT EINEM ZITRAT-GRÜPPE FÜR DIE PRODUKTION VON ANTIKÖRPERN WELCHE EINE AFFINITÄT HABEN FÜR TRI-PHOSPHORILIERTE NUCLEOSIDEN
USE OF NUCLOEOSIDE DERIVATIVES COMPRISING A CITRATE GROUP FOR PRODUCING ANTIBODIES HAVING AFFINITY FOR TRIPHOSPHORYLATED NUCLEOSIDES AND USES THEREOF

(30) Priorité: 05.03.2002 FR 0202782
(43) Date de publication de la demande: 12.01.2005
(73) Titulaire: CENTRE NATIONAL DE LA RECHERCHE SCIENTIFIQUE (CNRS), 75794 Paris Cedex 16 (FR)
(72) Inventeur: CUPO, Anny, F-06160 Juan-Les-Pins (FR); Le Saint, Cécile, F-06220 Vallauris (FR); Vincent, Jean-Pierre, F-06800 Cagnes-sur-Mer (FR); AKEB, Fatima, F-06100 NICE (FR); Duval, Danièle Les Jardins de Gairaut,, F-06100 NICE (FR); GUEDJ, Roger, F-06230 Villefranche-sur-Mer (FR)
(74) Mandataire: Breese, Pierre
(86) Numéro de dépôt international: PCT/FR2003/000708
(87) Numéro de publication internationale: WO 2003/075009

(56) Documents cités:
- US-A- 5 380 825
- WEAVER R ET AL: "Isosteres of nucleoside triphosphates" BIOORGANIC & MEDICINAL CHEMISTRY LETTERS, OXFORD, GB, vol. 6, no. 20, 22 octobre 1996 (1996-10-22), pages 2405-2410, XP004135847 ISSN: 0960-894X cité dans la demande
- BROSSETTE T ET AL: "Synthesis of polyphosphorylated AZT derivatives for the development of specific enzyme immunoassays" JOURNAL OF ORGANIC CHEMISTRY, AMERICAN CHEMICAL SOCIETY. EASTON, US, vol. 64, 1999, pages 5083-5090, XP002173201 ISSN: 0022-3263
- AKEB FATIMA ET AL: "The production and evaluation of antibodies for enzyme immunoassay of AZTTP." NUCLEOSIDES NUCLEOTIDES & NUCLEIC ACIDS, vol. 20, no. 3, mars 2001 (2001-03), pages 243-250, XP001109635 ISSN: 1525-7770

## Description

La présente invention concerne un immunogène comportant un isostère de nucléoside triphosphate dans lequel le groupement phosphate a été remplacé par un groupement citrate, son utilisation pour la production d'anticorps et un procédé d'obtention d'un anticorps.

### Art antérieur

Dans le cadre de la présente invention on entend par :
- nucléoside naturel : une molécule constituée par l'association d'une base purique (adénine ou guanine) ou pyrimidique (cytosine, uracile et thymine) avec un résidu pentose (béta-D-ribofuranose ou béta-D-deoxyribofuranose).
- analogue nucléosidique (ddNs): toute molécule composée d'une base modifiée ou non, couplée à un ribose ou à un analogue de celui-ci.

Des tels analogues sont par exemple décrits dans le document :Périgaud, C. et al. Nucleosides and Nucleotides 1992, vol. 11(2-4), pages 903-945).

Ainsi par rapport à un nucléoside naturel, un analogue peut comporter des modifications de la molécule du ribose, comme par exemple celles mentionnées page 607 du document précité, telles que la substitution d'un ou plusieurs atomes, le déplacement de la liaison entre la base et le sucre, des inversions anomériques (béta-alpha), l'addition de fonctions diverses, l'inversion, substitution ou élimination de groupements hydroxyles, la modification de la taille du cycle (pyranose), l'inversion de la configuration (D-L) ou encore la rupture du cycle (acyclonucléosides) ou des modifications de la base hétérocyclique comme par exemple celles indiquées page 908 dudit document, pour les analogues :Fura, FdUrd, Thi-Gua, 6-MP, AraC ou Fludarabine phosphate.

Dans le cadre de la présente invention, le terme nucléoside employé dans le texte désigne indistinctement un nucléoside naturel ou un analogue nucléosidique tels que ci-dessus définis.

Certains nucléosides sont couramment employés en tant qu'agents anti-viraux ou en tant qu'agents anti-cancéreux et on connaît qu'ils doivent être métabolisés en leur forme triphosphorylée pour pouvoir exercer une activité inhibitrice antivirale ou anticancéreuse.

Dans le cadre des traitements anti-viraux et notamment dans les thérapies anti-VIH, il est important de quantifier ces formes triphosphorylées des analogues nucléosidiques.

Ainsi, dans le but de quantifier la forme active des analogues nucléosidiques, il est essentiel d'utiliser des méthodes de dosage sensibles et spécifiques desdits nucléosides triphosphorylés, ci-après désignés ddNTPs.

Un des facteurs déterminants de l'échec thérapeutique des traitements anti-VIH s'expliquerait par une altération du métabolisme cellulaire pour les nucléosides(ddNs), ce qui entraînerait une concentration insuffisante de la molécule active dans le milieu intracellulaire, cause majeure des échappements thérapeutiques. Par conséquent, un des moyens d'appréhender l'échappement thérapeutique serait la détermination de la concentration intracellulaire de ces molécules (nucléosides triphosphorylés et antiprotéases) par dosages immunologiques. Le suivi thérapeutique des patients ou TDM (Therapeutic Drug Monitoring) peut être alors envisagé pour optimiser l'administration de ces molécules en proposant des traitements personnalisés mais aussi permettre d'évaluer les associations.

Le catabolisme très important des antiprotéases par les enzymes hépatiques et intestinales ainsi que les mécanismes d'efflux conduisent à administrer des doses d'antiviraux très importantes aux malades pouvant générer des effets secondaires redoutables.

En ce qui concerne les analogues nucléosidiques, le problème est différent. Les doses administrées sont là aussi importantes car imposées par la pénétration limitée du nucléoside précurseur dans les cellules. Les différents métabolites phosphorylés du médicament affecteront tout aussi bien les polymérases virales que cellulaires. La toxicité des catabolites du médicament viendra se surajouter à la non-spécificité des analogues nucléosidiques. Dans ce contexte, le traitement anti-VIH, à cause des quantités élevées d'antiviraux administrées aux malades, affectera un grand nombre de compartiments cellulaires y compris ceux qui sont réservés à la différentiation des cellules souches induisant ainsi les effets secondaires qui rendent les traitements anti-VIH si contraignants et inconfortables.

Ainsi les taux plasmatiques et intracellulaires des molécules anti-VIH, utilisées le plus souvent en association, sont le reflet direct du métabolisme de l'organisme entier. La bonne connaissance des taux plasmatiques et intracellulaires des molécules anti-VIH est essentielle et diffère d'un individu à l'autre et d'un moment à l'autre de la maladie.

La mesure de ces paramètres permettrait aux cliniciens de déterminer les conséquences des associations, d'en optimiser leur utilisation et d'en adapter la posologie en fonction du malade et de l'évolution de sa maladie. Il est essentiel pour le clinicien d'avoir accès à la mesure du contenu intracellulaire qui est en étroite relation avec la concentration plasmatique et la charge virale. Cette dernière a un impact direct sur l'échappement thérapeutique.

Des méthodes de dosages intracellulaires des dérivés nucléosidiques ont été développées, Robbins, BL, et al. (1996) "Quantification of intracellular zidovudine phosphates by use of combined cartridge-radioimmunoassay methodology". Antimicrob. Agents Chemother. 40, 2651-2654.; Goujon, L. et al. (1998) "Monitoring of intracellular levels of 5'-monophosphate-AZT using an enzyme immunoassay". J. Immunol. Methods, 218, 19-30.; Robbins, BL. et al. (1998) A. "Development of a new cartridge radioimmunoassay for determination of intracellular levels of lamivudine triphosphate in the peripheral blood mononuclear cells of human immunodeficiency virus) infected patients". Antimicrob. Agents Chemother. 42, 2656-2660.).

Mais ces méthodes n'offrent pas des performance suffisantes du point de vue de la sensibilité du test, nécessaires notamment pour détecter les faibles concentrations des analogues nucléosidiques présents dans les compartiments cellulaires et disponibles dans des échantillons de faible taille.

On décrit ci-dessous, dans la partie exposé de l'invention des méthodes de quantification des analogues nucléosidiques , permettant de pallier les inconvénients des dosages existants desdits composés, qui sont utilisés par exemple, en tant que composés antiviraux

On décrit également, ci-dessous dans la partie exposé de l'invention des méthodes de quantification des nucléosides au moyen de dosages immunologiques présentant de nombreux avantages en termes de sensibilité et de rapidité car tant le temps de préparation des échantillons comme celui du dosage lui-même sont considérablement réduits.

Ils sont de plus, applicables à un grand nombre d'échantillons de taille réduite, tant en volume qu'en nombre de cellules. Ils constituent une méthode de quantification réalisable en mélange complexe, sensible, adaptée aux concentrations intracellulaires faibles, rapide et adaptée au suivi de patients en milieu hospitalier.

Il existe peu de travaux prenant en compte le métabolisme intracellulaire des nucléosides en raison de l'instabilité des dérivés triphosphorylés du fait que les liaisons pyrophosphates sont sensibles à l'hydrolyse et rapidement dégradées.

Bien qu'il existe pour les nucléosides une méthode de mesure par HPLC et EC (électrophorèse capillaire). Elle reste cependant difficilement applicable pour le suivi thérapeutique des patients. Cette dernière nécessitant une préparation minutieuse des échantillons, implique un temps d'analyse long et un volume sanguin important pour permettre le dosage des taux intracellulaires faibles. D'autre part, les coûts de l'investissement nécessaire à l'achat de l'appareillage et du fonctionnement de ce dernier, tels que les solvants ou les colonnes, représentent un inconvénient majeur pour les laboratoires et les hôpitaux.

En ce qui concerne les dérivés phosphorylés des analogues nucléosidiques, il n'existe pas de méthode de dosage chromatographique. En revanche, il existe des méthodes immunologiques mais elles sont indirectes et nécessitent plusieurs étapes, entre autres, la purification des métabolites phosphorylés, la déphosphorylation enzymatique, ou encore l'élimination des sels et réactifs.(Robbins, BL, et al. (1996) "Quantification of intracellular zidovudine phosphates by use of combined cartridge-radioimmunoassay methodology". Antimicrob. Agents Chemother. 40, 2651-2654.; Goujon, L. et al. (1998) "Monitoring of intracellular levels of 5'-monophosphate-AZT using an enzyme immunoassay". J. Immunol. Methods, 218, 19-30.; Robbins, BL. et al. (1998) A. "Development of a new cartridge radioimmunoassay for determination of intracellular levels of lamivudine triphosphate in the peripheral blood mononuclear cells of human immunodeficiency virus) infected patients". Antimicrob. Agents Chemother. 42, 2656-2660).

Par ailleurs, certaines méthodes envisagées pour obtenir de tels anticorps, par exemple, des anticorps dirigés contre l'AZT-TP, en utilisant comme immunogène un dérivé de l'AZT-TP dans lequel on a substitué les liaisons anhydride phosphorique par des liaisons méthylènephosphonate (ou P-CH₂-P), le groupement méthylène-bis-phosphonate (P-CH₂-P-CH₂-P) possède des caractéristiques structurales voisines de celles du groupement pyrophosphate (P-O-P-O-P) puisque l'angle des liaisons anhydride phosphorique (P-O-P) est de 130°, celui de la liaison méthylènephosphonate est de 117°; la longueur des liaisons P-O est de 1,61Å, et celle des liaisons P-C est de 1,79 Å. (Saady et al. (1995), "First synthesis of fully deprotected diimidotriphosphoric acid and derivatives designed for the synthesis of « PNPNP » nucleotides and dinucleotides" .J.Org.Chem. vol 60, pp 3685-3691) (Brosette et al. (1999), "Synthesis of polyphosphorylated AZT Derivatives for the Development of specific enzyme immunoassays" J. Org. Chem. vol 64, pp 5083-5090).

Cependant, l'affinité des différents anticorps dirigés contre cet analogue méthylène-bis-phosphonate de l'AZT-TP obtenus, n'est pas suffisante vis-à-vis de la molécule d'AZT-TP à doser pour permettre sa quantification, d'autant plus que les quantités intracellulaires des nucléotides sont extrêmement faibles et que le volume de l'échantillon à doser est également réduit.

La préparation d'anticorps pour le dosage des dérivés ddNTPs, les reconnaissant avec une affinité suffisante pour permettre leur quantification intracellulaire est également décrit dans la partie exposé de l'invention.

De plus, ces anticorps ne devront pas reconnaître les nucléotides endogènes.

### Exposé de l'invention

Pour atteindre ce but, la demanderesse a mis au point des immunogènes, susceptibles d'induire une réponse immune chez des mammifères, préparés à partir d'analogues de nucléosides présentant non seulement une analogie structurale, mais également une analogie électronique avec la molécule à doser pour l'immunisation de mammifères, appelés ci-après des isostères.

De tels isostères de nucléosides triphosphates ont déjà été décrits, par exemple pour la conception d'un inhibiteur de la transcriptase inverse. (Weaver, R and Gilbert, IH. (1997) "The design and synthesis of nucleoside triphosphate isostere as potential inhibitors of HIV reverse transcriptase". Tetrahedron, vol.53, 5537-5562.; Weaver, R, et al. (1996) "Isosteres of nucleoside triphosphate"s. Bioorg. Med. Chem. Lett. Vol. 6, 2405-2410.

Un premier objet de la présente invention est donc l'utilisation desdits isostères de nucléosides triphosphates de préférence, des isostères dans lesquels leur groupement phosphate a été remplacé par le groupement citrate, pour la préparation d'immunogènes par couplage desdits isostères sur des molécules porteuses.

L'avantage de cette approche consiste en ce que l'on obtient une meilleure détection de la molécule triphosphorylée car l'anticorps a été produit contre une molécule isostère présentant des analogies structurales et électroniques avec ledit dérivé triphosphorylé et ressemblant d'avantage à celui-ci.

L'immunisation de cellules immunocompétentes de vertébrés, notamment de mammifères, a en effet, permis d'obtenir des anticorps ayant une affinité adaptée aux dosages des dérivés ddNTPs y compris lorsque ceux-ci ne sont présents qu'à des très faibles doses.

L'invention concerne donc un immunogène comportant isotère de nucléoside triphosphate dans lequel le groupement phosphate a été remplacé par un groupement citrate couplé à une molécule porteuse.

De préférence ledit nucléoside ou analogue est choisi parmi le groupe comprenant: Acyclovir, Adénosine, S-Adenosyl-L-methionine, 2',3'-didéoxyadénosione (ddA), 2',3'-didehydro-2',3'-didéoxythymidine (d4T), 2',3'-dideoxy-3'-thiacytidine (3TC), 3'- Azido-3'-deoxythymidine (AZT), Carbovir, Cordycepine, Cytidine, Cytosine-b-D-arabinoside, Deoxycytidine, Deoxytubercidine, 2'-Deoxyuridine, Formycine A, Formycine B, Ganciclovir, Guanosine, Inosine, Puromycine, Ribavirine, Sangivamycine, Thymidine, Tubercidine, Uridine, Abacavir, 3-fluoro-2',3'-dideoxythymidine (FLT), Fura, FdUrd, Thi-Gua, 6-MP, AraC ou Fludarabine phosphate.

Ainsi que des produits anti-cancéreux antimétaboliques tels que :
- Les analogues des bases puriques : 6-mercaptopurine, cladribine, fludarabine.
- Les analogues des bases pyrimidiques : 5-fluoro-uracile, cytarabine, gemcitabine, Tenofovir.

En tant que molécule porteuse on peut utiliser toute molécule de haut poids moléculaire, de préférence comprise entre 50000 et 500000 daltons, capable d'induire une réponse immune. De préférence, la molécule porteuse est choisie parmi une protéine, un polypeptide phylogénétiquement éloignés de l'espèce animale utilisée pour l'immunisation, ou un polysaccharide.

Tout préférentiellement la molécule porteuse est une protéine choisie parmi le groupe comprenant l'ovalbumine, le sérum albumine bovine, l'hémocyanine de patelle, la thyroglobuline, des immunoglobulines, la caséine, l'hémoglobine, des toxines bactériennes, telles que la sous-unité B de la toxine du choléra, la toxine tétanique, la toxine diphtérique, de lectines telles que la sous-unité B de la ricine.

L'invention a également pour objet l'utilisation des immunogènes, ci-dessus définis pour obtenir des anticorps spécifique des nu cléosides-citrate ou des dérivés nucléosides-citrate utilisés pour l'immunisation.

L'invention a également pour objet l'utilisation des immunogènes ci-dessus définis pour la préparation d'anticorps, ayant une affinité pour un dérivé ddNTP.

Un autre objet de l'invention concerne un procédé d'obtention d'un anticorps ayant une affinité pour ledit dérivé ddNTP.

Il peut s'agir d'anticorps polyclonaux, un tel premier procédé comprend alors les étapes suivantes :
a) l'immunisation d'un animal non humain, avec au moins un immunogène de l'invention, lequel immunogène comporte un dérivé citrate de nucléoside couplé à une molécule porteuses, .
b) la purification des anticorps polyclonaux produits.

L'animal immunisé à l'étape (a) est un mammifère non humain, un rongeur notamment un rat ou une souris, une chèvre, un lapin ou une poule et la purification des anticorps de l'étape (b) peut s'effectuer à partir du sang de l'animal immunisé ou à partir du jaune d'oeuf dans le cas de la poule.

Il peut s'agir d'anticorps monoclonaux, un tel second procédé comprend alors les étapes suivantes :
a) l'immunisation de cellules immunocompétentes d'un animal non humain, avec au moins un immunogène de l'invention,
b) l'immortalisation des cellules sécrétant des anticorps ayant une affinité pour ledit dérivé ddNTP au moyen d'une fusion cellulaire des cellules de l'animal immunisées, isolées à partir de la rate ou des ganglions lymphatiques- avec une lignée cellulaire de fusion,
c) l'identification et la sélection des clones hybrides immortalisés, hybridomes, sécrétant lesdits anticorps ayant une affinité pour ledit dérivé ddNTP,
d) la purification des anticorps produits par lesdits hybridomes.

Dans ce second procédé, les cellules immunocompétentes d'un animal, par exemple, des cellules de la rate ou des ganglions lymphatiques dudit animal, sont immunisées soit *in vivo* soit *in vitro.*

De préférence, la lignée de fusion, utilisée à l'étape (b) du second procédé est choisie parmi une lignée myélomateuse de souris, une lignée myélomateuse de rat ou encore des lignées permettant la préparation des hétérohybrides.

La présente invention permet d'obtenir des hybridomes ainsi obtenus, susceptibles de synthétiser et de sécréter des anticorps monoclonaux ayant une affinité par un dérivé ddNTP.

La présente invention permet d'obtenir anticorps spécifique des isostères nucléoside-citrate et en particulier un anticorps anti-nucléoside-citrate reconnaissant au moins un immunogène de l'invention.

La présente invention permet d'obtenir un anticorps ayant une affinité pour un dérivé ddNTP obtenu par l'un quelconque des procédés d'obtention d'anticorps décrits ci-dessus.

De préférence, l'affinité des anticorps par le dérivé ddTP contre lequel ils ont été préparés, définie par son IC50, est supérieure à 10⁻⁷, tout préférentiellement supérieure à 10⁻⁹ et tout préférentiellement supérieure à 10⁻¹⁰.

L'IC 50 est définie par la concentration d'antigène non marqué capable d'inhiber 50 % de la liaison antigène-anticorps. L'IC 50 est un bon reflet de l'affinité. L'affinité est déterminée par la méthode de Scatchard.

La présente invention permet d'obtenir également un fragment d'anticorps ayant une affinité pour un dérivé ddNTP comprenant au moins une partie variable d'une molécule d'un anticorps ayant la même affinité pour ledit dérivé ddNTP que ladite molécule d'anticorps non fragmentée.

De tels fragments sont par exemple des fragments Fv, Fab, Fab', F(ab)₂, F(ab')₂, qui peuvent être obtenus par digestions enzymatiques ou par clivages chimiques à partir des anticorps non-fragmentés, par des techniques connues de l'homme du métier.

La présente invention permet d'obtenir des immunoglobulines recombinantes, leurs chaînes lourdes et/ou legères ou des fragments de celles-ci que l'on peut obtenir en recombinant des acides nucléiques isolés codant pour les chaînes lourdes et/ou légères ou des fragments fonctionnels de celles-ci, des anticorps monoclonaux décrits ci-dessus, en exprimant lesdites acides nucléiques recombinés dans un hôte cellulaire procaryote tel qu'E.coli ou dans un hôte eucaryote tel que les levures ou encore dans une cellule de mammifère.

La présente invention permet d'obtenir un procédé de dosage immunologique de dérivés triphosphorylés nucléosidiques.

Ces dérivés triphosphorylés sont formés notamment lors du métabolisme des nucléosides utilisés dans les thérapies anti-virales et en particulier des thérapies anti-VIH, mais aussi lors des traitements anticancéreux.

Des tels dérivés triphosphorylés sont par exemple des dérivés triphosphorylés d'AZT, F3dThd, IdUrd, AraA, virazole, acyclovir, DHPG ou ganciclovir, d4T, 3TC, ddA, et le dérivé diphosphorylé du Tenofovir.

Ce procédé de dosage immunologique du dérivé nucléosidique triphosphorylé au moyen des anticorps ayant une affinité pour ledit dérivé permet le dosage d'un grand nombre d'échantillons. Grâce à sa sensibilité, il ne nécessite pas un volume d'échantillon important. En effet, la Demanderesse a pu quantifier in vitro le dérivé triphosphorylé ddATP dans 10⁶ à 4x10⁶ CMSP préalablement incubées avec des concentrations variables de ddI (10⁻⁷ à 10⁻⁹M).

Selon un mode de réalisation, dans la mesure où ces anticorps détectent le dérivé triphosphorylée, la méthode de dosage de nucléosides triphosphorylés permet de s'affranchir des étapes supplémentaires nécessaires dans les autres méthodes, telles que la dégradation enzymatique ou la purification sur cartouche Sep-Pak C18

Selon un mode de mise en oeuvre, le procédé de dosage d'un dérivé ddNTP selon l'invention comporte les étapes suivantes :
a) la mise en contact d'au moins un anticorps ayant une affinité pour un dérivé ddNTP de l'invention, avec un échantillon susceptible de contenir ledit nucléoside triphosphate ou un analogue de celui-ci, éventuellement en présence d'un traceur marqué.
b) la séparation du complexe formé par l'anticorps ayant une affinité pour ledit dérivé ddNTP lié audit dérivé ddNTP dudit dérivé ddNTP libre,
c) la mesure du rapport entre la quantité de dérivé ddNTP lié par l'anticorps versus le dérivé ddNTP non lié.

De préférence le traceur est choisi parmi un dérivé du nucléoside à détecter comportant un motif susceptible d'être marqué, choisi parmi un motif comprenant une tyrosine, une histidine ou tout acide aminé ou groupement possédant une fonction amide, de préférence susceptible d'introduire un élément radioactif ou susceptible de l'être, tel que par exemple le réactif de Bolton-Hunter.

Un traceur particulièrement préféré comporte un motif dipeptidique Tyr-Lys ou un motif Lys-Tyr.

Le traceur peut être marqué au moyen d'une enzyme, un chromophore, un colorant, un fluorophore, une particule, comme une particule métallique ou magnétique, un pigment ou un atome radioactif.

La présente invention permet d'utiliser un procédé de dosage d'un dérivé ddNTP selon l'invention pour le dosage d'un dérivé ddNTP dans un échantillon biologique.

Selon un premier mode de réalisation, le dosage du dérivé ddNTP est effectué dans un fluide biologique.

Ledit fluide biologique peut être du sang, du plasma, du sérum, du liquide céphalorachidien (LCR), des urines, de la lymphe, de la salive.

Selon un deuxième mode de réalisation, le dosage du dérivé ddNTP est effectué dans un échantillon biologique comprenant des cellules.

Lesdites cellules sont des lymphocytes, des monocytes périphériques, des cellules de Langehrans, des cellules dendritiques, des cellules présentatrices, des cellules souches, des adipocytes, des cellules immunitaires, éventuellement triées, telles que des cellules CD4⁺ ou CD8⁺.

Selon un troisième mode de réalisation, le dosage du dérivé ddNTP est un dosage de type histologique sur une coupe de tissu, tel que le tissu ganglionnaire ou sur un étalement de cellules. La visualisation de l'anticorps direct ou indirectement marqué permet alors d'observer la localisation et la distribution du dérivé ddNTP dans le tissu cible.

La révélation des antiviraux se fait alors après fixation des cellules par différents réactifs visant à immobiliser les molécules à révéler au site cellulaire où ils sont localisés. Ces fixateurs peuvent être de plusieurs types comme les vapeurs d'anhydride succinique, le paraformaldéhyde, la glutaraldéhyde ou les carbodiimides.

Le procédé de dosage d'un dérivé ddNTP décrit est applicable au dosage desdits dérivés chez un patient ayant fait l'objet d'une thérapie antivirale avec des analogues nucléosidiques, notamment dans le cadre des thérapies anti-VIH, mais il peut être également utilisé pour le dosage d'un dérivé ddNTP formé chez un individu soumis à des traitements anti-cancéreux qui utilisent aussi des dérivés nucléosidiques en tant qu'agents actifs.

L'invention est illustrée à l'aide des travaux expérimentaux qui décrivent la mise en oeuvre de l'invention pour un analogue nucléosidique, le ddATP.

Bien entendu, il est possible d'adapter cette méthodologie pour la production d'anticorps susceptibles de détecter n'importe quel analogue nucléosidique triphosphorylé et de concevoir un procédé de dosage immunologique les mettant en oeuvre.

Cet exemple, non limitatif, de mise en oeuvre de l'invention, concernant le dérivé ddATP et détaillé ci-après et à l'aide des figures en annexe dans lesquelles :
- la figure 1 représente la détermination de la nature du matériel immunoréactif présent dans le surnageant de culture par chromatographie sur cartouche anionique et détection radioimmunologique,
- la figure 2 représente la détermination de la nature du matériel immunoréactif présent dans les cellules, purifié par chromatographie sur cartouche anionique et détecté par RIA,
- la figure 3 montre la caractérisation du matériel immunoréactif présent dans un extrait cellulaire par chromatographie sur cartouche anionique Sep Pak et détection radioimmunologique,
- la figure 4 montre le profil d'immunoréactivité des fractions correspondant probablement au ddAMP et ddADP avant et après déphosphorylation.
- la figure 5 montre l'analogue ddATP et un isostère de celui-ci dans lequel le groupement triphosphate est substitué par le groupement citrate pour conduire au 5'-O-(3,4-dicarboxy-3-hydroxy)butanoate-2',3'-didésoxyadénosine.
- la figure 6 illustre la modélisation moléculaire qui confirme que la molécule résultante du couplage de l'acide 2-hydroxy-2,3-allyloxycarbonylbutanoïque sur la position 5' de la ddA possède des caractéristiques structurales et électroniques voisines de celles du ddATP.
- la figure 7 montre un schéma de l'obtention du diester allylique de l'acide citrique 5 en quatre étapes.
- la figure 8 montre le couplage de la ddA à l'acide 2-hydroxy-2,3-allyloxycarbonylbutanoïque en présence de dicyclohexylcarbodiimide (DCC) et de 1-hydroxy-7-azabenzotriazole (HOAt) dans le tétrahydrofurane pour la préparation du 5'-O-((3,4-diallyloxycarboxyl)-3-hydroxy)butanoate-2',3'-didésoxyadénosine.

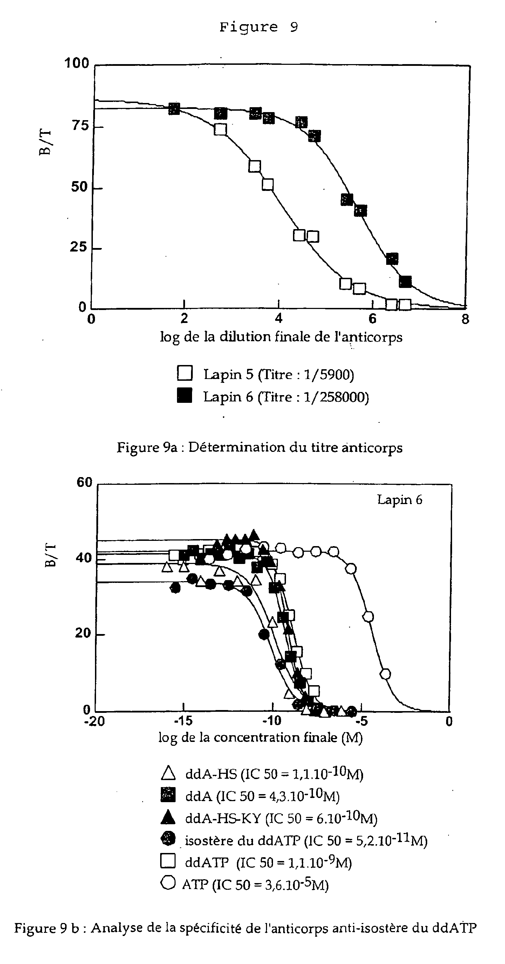

- la figure 9 illustre les caractéristiques des anticorps anti-isostère du ddATP en test RIA avec le traceur dd-A-HS-(Y*-K). La figure 9a illustre le rapport antigène lié versus antigène libre en fonctions des dilutions finales des anticorps et permet de déterminer le titre desdits anticorps. La figure 9b montre l'analyse de la spécificité de l'anticorps anti-isostère du ddATP.

### PARTIE EXPERIMENTALE

Dans le cadre des travaux expérimentaux réalisés lors de la mise en oeuvre de l'invention, la demanderesse a mis au point un dosage du 2',3'-didésoxyadénosine-5'-triphosphate ou ddATP, métabolite actif de la ddI utilisée comme agent antiviral, en produisant des anticorps dirigés contre une molécule isostère, dérivée du ddATP, possédant des analogies structurales et électroniques avec celle-ci.

### I. Préparation du dérivé 5'-O-(3,4-dicarboxy-3-hydroxy)butanoate-2',3'-didésoxyadénosine.

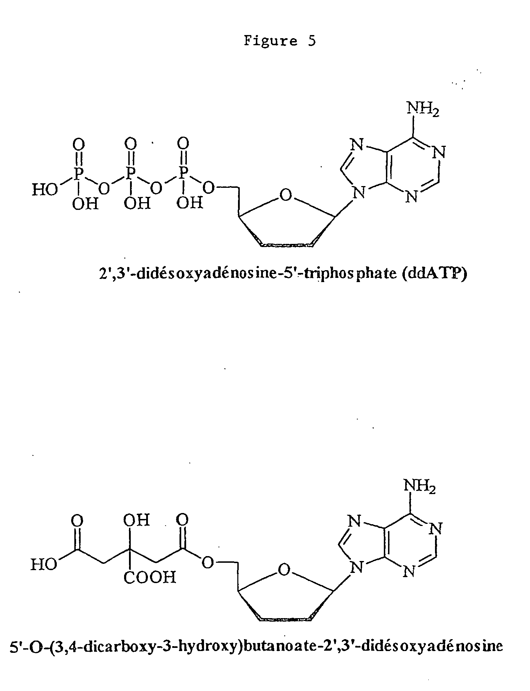

Dans cet analogue du ddATP, le groupement triphosphate est substitué par le groupement citrate pour conduire au 5'-O-(3,4-dicarboxy-3-hydroxy)butanoate-2',3'-didésoxyadénosine (Figure 5)

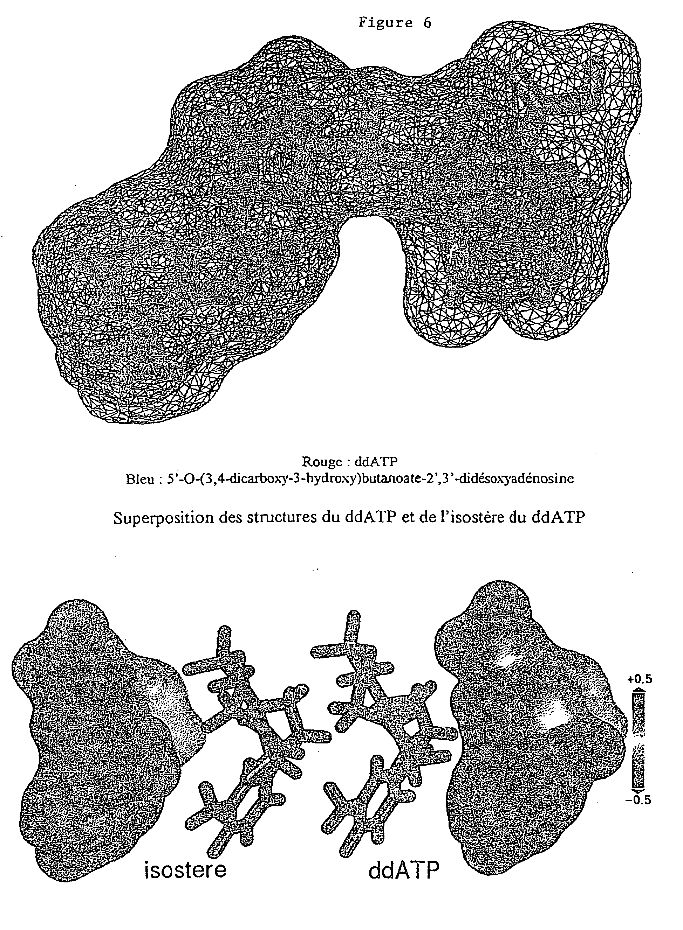

La modélisation moléculaire confirme que la molécule résultante du couplage de l'acide 2-hydroxy-2,3-allyloxycarbonylbutanoïque sur la position 5' de la ddA possède des caractéristiques structurales et électroniques voisines de celles du ddATP. (Figure 6)

La synthèse de 5'-O-(3,4-dicarboxy-3-hydroxy)butanoate-2',3'-didésoxyadénosine requiert d'abord la protection de deux des fonctions acides carboxyliques de l'acide citrique sous forme d'ester allylique.

La synthèse de l'acide 2-hydroxy-2,3-allyloxycarbonylbutanoïque est réalisée selon la méthode décrite par Weaver, R. et Gilbert, IH. (Tetrahedron, (1997), 53, 5537-5562. *The design and synthesis of nucleoside triphosphate isosteres* as *potential inhibitors of HIV reverse transcriptase).*

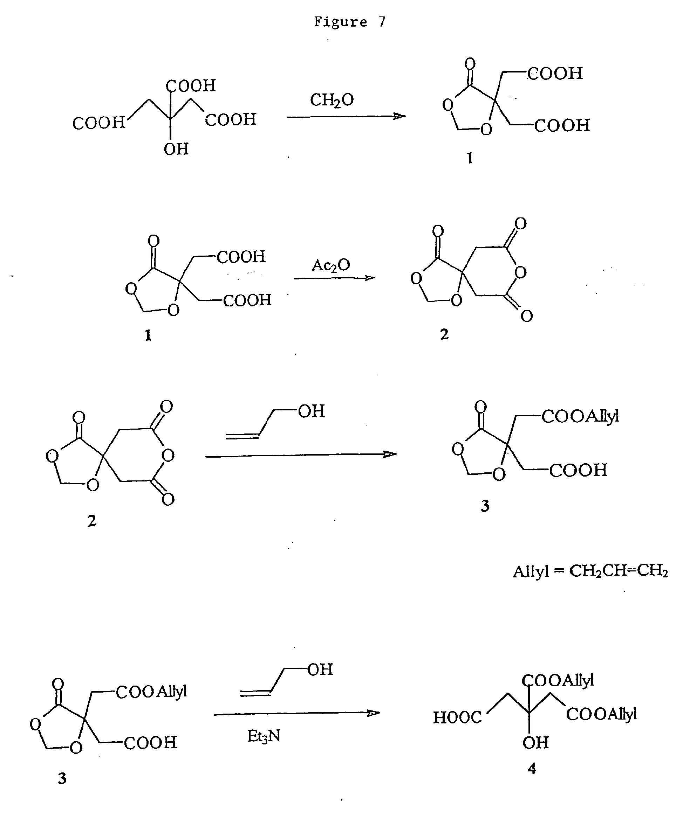

La préparation de l'acide 2-hydroxy-2,3-allyloxycarbonylbutanoïque est réalisée en 4 étapes :
a) la formation de l'oxalactone 1 par traitement de l'acide citrique avec le paraformaldéhyde,
b) la cyclisation du diacide 2 en anhydride 3 par échange avec l'anhydride acétique.
c) l'obtention du monoester allylique 4 par ouverture de l'anhydride 3 par l'alcool allylique.
d) l'obtention du diester allylique de l'acide citrique 5 par ouverture de la lactone et hydrolyse du méthylène dioxy (Figure 7).

Les conditions de couplage de la ddA à l'acide 2-hydroxy-2,3-allyloxycarbonylbutanoïque décrites dans l'article de Weaver R. (1997) et al. précité ne sont pas satisfaisantes, car le rendement de couplage est très faible et s'accompagne de la dégradation partielle du produit à cause de la déshydratation du β-hydroxyester en milieu basique.

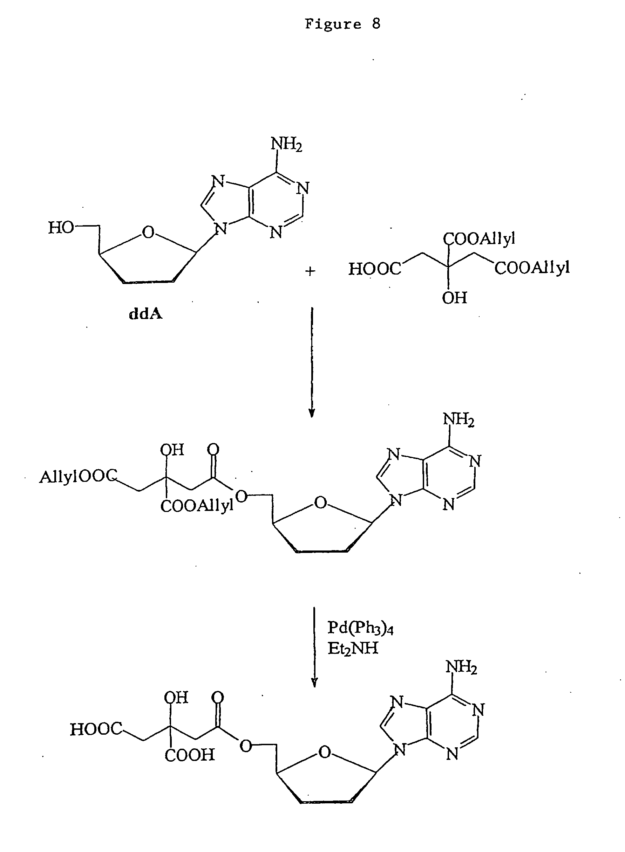

La demanderesse a ainsi amélioré les conditions de couplage de la ddA à l'acide 2-hydroxy-2,3-allyloxycarbonylbutanoïque en effectuant ce couplage en présence de dicyclohexylcarbodiimide (DCC) et de 1-hydroxy-7-azabenzotriazole (HOAt) dans le tétrahydrofurane pour conduire à la formation de 5'-O-((3,4-diallyloxycarboxyl)-3-hydroxy)butanoate-2',3'-didésoxyadénosine (Figure 8).

Dans ces conditions, on élimine la réaction de déshydratation.

Ce couplage est effectué en présence de pyridine pour des raisons de solubilité de la ddA. La déprotection des fonctions acides carboxyliques est effectuée en présence de tétrakis(triphénylphosphine) palladium (0) et de diéthylamine dans du chlorure de méthylène.

Le 5'-O-(3,4-dicarboxy-3-hydroxy)butanoate-2',3'-didésoxyadénosine est obtenu avec un rendement quantitatif après lavage de la phase aqueuse puis recristallisation.

Le même protocole a été utilisé pour l'AZT-citrate, le d4T-Citrate et le 3TC-Citrate.

### II. Préparation d'immunogènes. Procédés de couplage ou activation de l'haptène et couplage à la protéine porteuse

Le couplage à une protéine porteuse implique l'activation d'un ou plusieurs groupements carboxylate de l'haptène. L'activation de ce carboxylate s'effectue soit par un chloroformiate d'alkyle (généralement le chloroformiate d'éthyle), soit par un chlorocarbonate d'alkyle soit un carbodiimide en présence de N-hydroxysuccinimide permettant d'augmenter le rendement de couplage à la protéine porteuse. Concernant les nucléosides possédant un groupement carboxylate, l'activation de ce dernier est réalisable avec les réactifs précédemment décrit. En revanche, avec un chloroformiate ou un chlorocarbonate d'alkyle, la réaction d'activation induit la libération d'acide chlorhydrique dans le milieu réactionnel. Or la liaison glycosidique des nucléosides est sensible au milieu acide. Il est donc important de piéger cet acide en ajoutant au milieu réactionnel une base comme par exemple la triéthylamine. (Kaul, S., et al. 1996. Specific radioimmunoassays for the measurement of stavudine in human plasma and urine. J Pharm Biomed Anal. 15:165-174. et Ferrua, B. et al. 1994. Measurement of the anti-HIV agent 2',3'-didehydro-2',3'-dideoxythymidine (D4T) by competitive ELISA. J Immunol Methods. 176:103-110.)

Cinq mg de 5'-O-(3,4-dicarboxy-3-didésoxy-3-hydroxy)butanoate-2',3'-adénosine (12 µmoles) sent solubilisés dans 320 µl de DMF anhydre. On ajoute 8 mg de dicyclohexylcarbodiimide (3,6 équivalent) et 5 mg de N-hydroxysuccinimide (3,6 équivalent). Le mélange réactionnel est agité à 4°C pendant 3 heures. On solubilise 15 mg de KLH dans 1 ml de tampon Tris 10 mM à pH 7,7. Puis on ajoute goutte-à-goutte la solution contenant l'ester activé à la solution de KLH préalablement refroidie dans la glace. Le mélange réactionnel est agité pendant 4 heures à 4°C. Cette solution est conservée à -20 °C.

Le même protocole a été utilisé pour l'AZT, le d4T et le 3TC.

Le rapport molaire entre molécule porteuse et haptène est un paramètre important qui permet l'obtention d'un conjugué où le taux d'incorporation de l'haptène est suffisamment élevé pour induire une réponse immunitaire maximale entraînant la stimulation et l'activation des clones immunocompétents. (Cupo A. et al. (1984) "Quantitation and localization of Met-enkephalin-Arg-Gly-Leu in rat brain using highly sensitive antibodies". Neuropeptides 4, 389-401.; Cupo A. et al. (1984) "A new immunological approach to the detection and the quantification of the Met5-enkephalin precursors in rat brain". Neuropeptides 4, 375-387.; Cupo et al. (1985) Detection of methionine-enkephaline at the 10-16 mole level. J. Neuroimmunology, 8 : 57-67.; Cupo A. et al. (1987) "A new immunization procedure for the obtention of anti-leucine enkephalin antibodies. Part I. Immunization procedure and physicochemical characteristics of antibodies". Neuropeptides 8, 207-219.) ; Cucumel K. et al. (1996) "Production and characterization of site- directed antibodies against dermorphin and dermorphin related peptides" Peptides 17, 973-982.; Garreau I., et al. (1997) "Radioimmunoassay against the C-part of W-hemorphin7 peptide : An alternative assay for Cathepsin D activity" Peptides 18, 293-300.).

### III Préparation d'anticorps polyclonaux

Les anticorps polyclonaux ont été produits chez le lapin.

Les immunsérums ont été obtenus par immunisation de 2 lapins albinos (Centre d'élevage des Combes, Ain, France).

### III.1 - Protocole d'immunisation.

Lors de l'immunisation primaire, l'immunogène est administré à l'animal par voie intradermique, mais on peut également l'administrer par voies sous-cutanée, intramusculaire ou intrapéritonéale à raison de 500 µg d'immunogènes par immunisation et par lapin dilué au demi avec de l'adjuvant complet de Freund.

Un mois après l'immunisation primaire, les immunisations ont été effectuées à raison de un rappel toutes les trois semaines sur une période de quatre mois. Les saignées ont été réalisées dix jours après chaque rappel dans un tube hépariné pour éviter la coagulation du sang. Après centrifugation, le plasma est recueilli et conservé à -20°C.

### III.2 Caractérisation et sélection des anticorps ayant une affinité pour les dérivés ddNTPs.

Les anticorps ayant une affinité pour les dérivés ddNTPs peuvent être identifiés par des techniques immunologiques, soit à l'aide de dosages immunoenzymatiques, soit des dosages radioimmunologiques (RIA).

Les anticorps obtenus ont été caractérisés par dosage radioimmunologique en utilisant comme traceur le 5'-O-hémisuccinate-2',3'-didésoxyadénosine couplé au dipeptide Tyrosyl-Lysine radiomarqué à l'iode 125.

La comparaison des structures de l'isostère du ddATP et de l'hémisuccinate de la ddA montre des analogies confirmées par la modélisation moléculaire. Cette analogie structurale a été démontrée expérimentalement par le fait que le dérivé ddA-HS-K-¹²⁵IY est parfaitement reconnu par les anticorps anti-isostère du ddATP, de même que le dérivé non radiomarqué.

La spécificité de ces anticorps a été étudiée vis-à-vis de différents analogues du ddATP. Les caractéristiques de ces anticorps sont exprimées en termes d'affinité ou de sensibilité (ou IC 50 (M)) et présentées dans la table 1 ci-dessous. L'IC 50 (M) correspond à la concentration d'antigène non marqué qui inhibe 50 % de la liaison antigène-anticorps.

**Table 1**

| **Analogues** | **IC50 (M) ± SEM** |
|---|---|
| ddATP | 1,1 ± 0,1.10⁻⁹ (n=22) |
| ddA | 4 ± 0,8.10⁻¹⁰ (n=22) |
| ddA-HS-K-Y | 6.10⁻¹⁰ |
| Isostère du ddATP | 5,2 ± 1,8.10⁻¹¹ ( n=3 ) |
| ATP | 3,6.10⁻⁵ |
| dA | 3.10⁻⁷ |
| Adénosine | 2,4.10⁻⁶ |
| Adénine | 9.10⁻⁶ |
| ddI | 2 ± 0,3.10⁻⁸ (n=10) |
| AMP | nI |
| ADP | nI |
| 3TC | 2.10⁻⁴ |
| ddC | nI |
| AZT | 2.10⁻² |
| d4T | 2.10⁻² |

| | |
|---|---|
| nI : non immunoréactif jusqu'à des concentrations de 10⁻³M. | |

La séparation par chromatographie anionique des différents métabolites phosphorylés issus du métabolisme cellulaire de la ddI (ddIMP, ddAMP, ddADP et ddATP) est réalisée sur cartouche anionique Sep Pak à l'aide d'un gradient par palier utilisant différentes concentrations de KCl (6 ml KCl 50 mM, 10 ml KCl 100 mM et 6 ml 250 mM).

Le dosage immunologique du ddATP implique la séparation de ce dernier des autres métabolites phosphorylés de la ddA à l'aide d'un gradient de KCl. Par conséquent, nous avons contrôlé que les concentrations de KCl utilisées et même bien supérieure (jusqu'à 2,5 M) n'inhibent pas l'interaction antigène-anticorps.

### IV. Préparation d'anticorps monoclonaux.

Des anticorps monoclonaux dirigés contre chaque analogue nucléosidique ont été préparés par fusion de cellules de la rate des animaux immunisées avec les immunogènes correspondants, puis immortalisation des cellules stimulées, sécrétant des anticorps, au moyen d'une fusion avec des lignées myélomateuses de souris. Cette fusion est suivie d'une sélection des clones hybrides sécrétant les anticorps qui présentent une affinité par ledit analogue nucléosidique.

Pour effectuer la fusion cellulaire on peut utiliser des lignées myélomateuses telles que X63ag 8, SP2, NS, Y3 , selon la technique décrite par Kohler G. et Milstein C. (1975) « Continuous cultures of fused cells secreting antibody of predefined specificity ». Nature. 1975 Aug 7;256(5517):495-7, ainsi que les protocoles décrits dans CUPO A. and KALDY P. (1987) "Monoclonal antiidiotypic antibodies which recognized the binding site of delta receptor : fine specificity of the antiidiotypic antibodies". in "Progress in opioids Research" J.W. Holaday, P.Y. Law and A. Herz eds. Nida (Rockville, Maryland, USA) pp 25-28. et CUPO A., et al.. (1992) "Monoclonal antiidiotypic antibodies against δ opioid receptors as an electron microscopy probe". Eur. J. Cell. Biol. 57, 273-284.

### V. Dosage intracellulaire du ddATP. Validation sur un modèle cellulaire in vitro.

Les anticorps ainsi obtenus ont été utilisés pour la mise en oeuvre de dosages immunologiques appliqués pour le dosage intracellulaire des formes actives des nucléosides, notamment dans les monocytes humains du sang périphérique ou ils sont présents à de très faibles concentrations.

Un certain nombre de contrôles ont été réalisés sur un modèle de cellules *in vitro.* Après séparation par gradient de densité sur Ficoll, des cellules monocytaires du sang ont été incubées avec différentes concentrations de ddI dans du RPMI (à raison de 5.10⁵ cellules par ml).

L'immunoréactivité, présente dans le surnageant, est exprimée en équivalents (éq.) ddI établis par rapport à une courbe de référence ddI et illustrée dans le tableau 2 ci-dessous. L'immunoréactivité ddATP correspond à la somme des immunoréactivités présentes dans les fractions 32-37 issues de la séparation du contenu intracellulaire sur cartouche anionique Sep Pak. Elle est exprimée en équivalents ddATP par rapport à une courbe de référence ddATP et illustrée également sur le tableau 2 ci-dessous.

**Tableau 2**

| **Conc. de ddI (M) incubées** | **Conc. en ddI du surnageant en éq. ddI (M) m±SEM** | **Contenu intracellulaire en éq. ddATP (pmoles/10⁶ cellules) (m±SEM)** |
|---|---|---|
| 1 ×10⁻⁴ | 1,30 × 10⁻⁴ 1,50 × 10⁻⁴ | 16,2 51,0 |
| 1 ×10⁻⁵ | 3,8 ± 0,34 × 10⁻⁵ (n=2) | 10,4 ± 0,6 (n=2) |
| 5 ×10⁻⁶ | 5,74.10⁻⁶ (n=1) | 1,8 n=1 |
| 1 ×10⁻⁷ | 2,74.10⁻⁷ (n=1) | 0,15 n=1 |

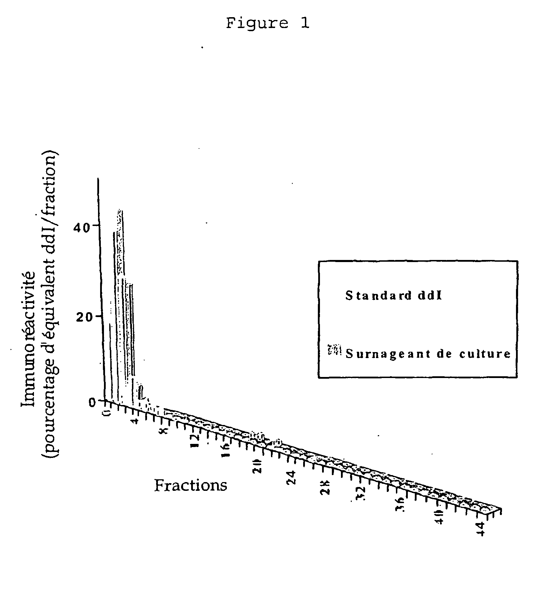

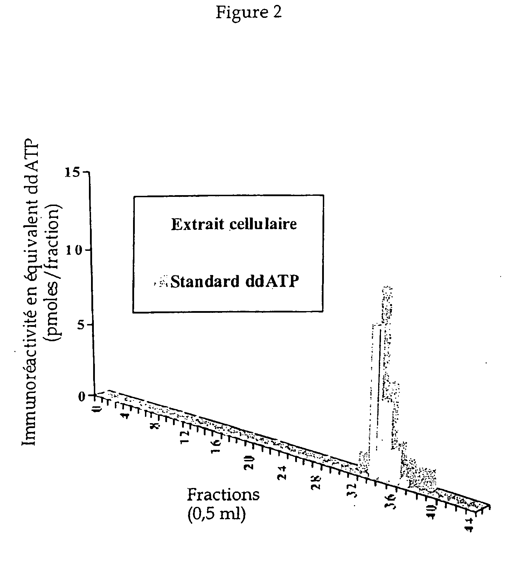

La nature du matériel immunoréactif présent dans le surnageant de culture et dans le milieu intracellulaire a été contrôlée par chromatographie sur cartouche anionique Sep Pak et détectée par dosage radioimmunologique. L'immunoanalyse de plusieurs surnageants de culture (n=4) montre que 100 % de l'immunoréactivité est retrouvée dans les fractions 1 à 5, correspondant au volume d'élution de la ddI et illustré dans la figure 1 en annexe. L'analyse des contenus intracellulaires montre que le matériel immunoréactif présent dans les cellules est élué au volume d'élution correspondant au ddATP avec un rendement de 92 % est illustré figure 2 en annexe. L'absence de matériel immunoréactif au temps de rétention de la ddI indique que la ddI n'est pas détectable dans les cellules.

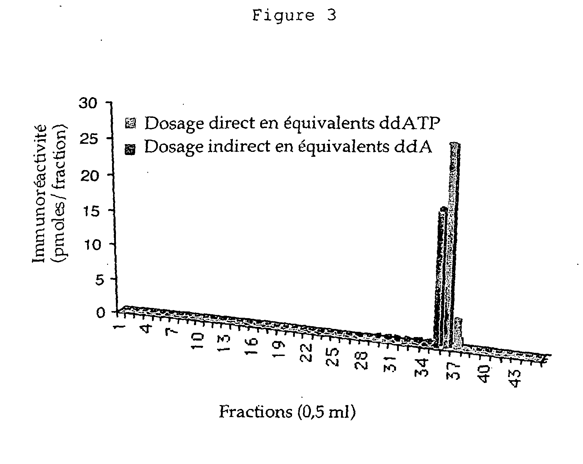

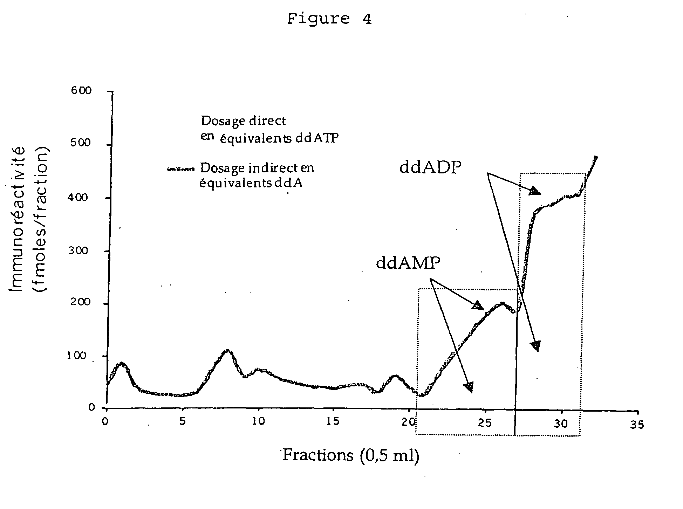

Pour détecter la présence éventuelle de ddAMP et de ddADP dans les contenus intracellulaires, il a été réalisé, après séparation sur cartouche anionique Sep Pak, un dosage après déphosphorylation des échantillons (dosage indirect exprimé en équivalents ddA). Ce dosage a été comparé à celui effectué sur les mêmes fractions non déphosphorylées (dosage direct en équivalents ddATP). Les résultats sont rapportés dans les figures 3 et 4 en annexe. Elles correspondent à l'analyse d'un extrait cellulaire obtenu après incubation des cellules avec une concentration de ddI de 1,5.10⁻⁴M.

Le ddATP est présent dans les fractions 32-37 correspondant au volume d'élution du ddATP standard. La quantité de ddATP détectée au niveau de ces fractions après déphosphorylation représente 93 % de la quantité de ddATP avant traitement.

Lorsque ces résultats sont retraités en modifiant l'échelle des immunoréactivités, au niveau des fractions précédant les fractions contenant le ddATP, on observe deux épaulements qui pourraient correspondre respectivement au ddAMP et au ddADP. En effet, les fractions 24 à 26 et les fractions 27 à 30 sont des fractions dont l'immunoréactivité est fortement augmentée après traitement à la phosphatase acide, indiquant qu'elles contiennent le motif ddA libérable par le traitement enzymatique. L'augmentation de l'immunoréactivité d'un facteur 5 pourrait traduire le facteur de croisement qui existe entre les analogues mono- et diphosphorylés par rapport au ddATP.

### VI. Dosage des nucléosides dans les échantillons biologiques. Applications cliniques.

Les prélèvements des échantillons sanguins de malades infectés par le VIH-1 et suivant un traitement comprenant la ddI ont été réalisés dans le service du Pr. P. Dellamonica à l'Hôpital L'Archet de Nice. Les prélèvements sanguins sont réalisés dans des tubes CPT à raison de 2 tubes de 4 ml pour les 4 patients faisant partie de cette étude. Pour chaque patient, le dosage de la ddI a été réalisé dans le plasma et sera exprimé en équivalents ddI. Le dosage intracellulaire de ddATP a été réalisé après chromatographie (sur cartouche anionique Sep Pak) des lysats cellulaires. Pour chaque fraction (500 µl), le contenu cellulaire en ddATP sera déterminé directement et exprimé en équivalents ddATP et après déphosphorylation et exprimé en équivalents ddA.

### VI.1. Dosages plasmatiques du ddATP

Les concentrations plasmatiques déterminées chez les 4 patients sont relativement comparables et varient de 2 à 6.10⁻⁶ M. Elles sont illustrées dans le tableau 3 ci-dessous.

**Tableau 3**

| **Patients** | **Conc. en éq. ddI (10⁻⁶ M)** | **Conc. en éq. ddI (µg/ml)** |
|---|---|---|
| BED | 6,10 | 1,44 |
| IPE | 1,95 | 0,46 |
| TRI | 3,20 | 0,76 |
| PRI | 2,95 | 0,70 |

### VI.2. Dosages intracellulaires du ddATP.

Les éléments figurés du sang sont isolés par centrifugation dans des tubes pour préparations cellulaires (Cell preparation tubes ou CPT, Vacutainer^{R}). Les cellules mononuclées sont lavées par 2 fois 15 ml de milieu RPMI, centrifugées et comptées, le dénombrement des cellules pour chacun des échantillons de l'étude est indiqué dans la table 4 ci-dessous, puis le culot cellulaire est congelé à -70°C.

**Table 4**

| **patient** | **Nombre de cellules par ml** |
|---|---|
| BED | 0,49.10⁶ |
| IPE | 0,98.10⁶ |
| TRI | 1,67.10⁶ |
| PRI | 1,35.10⁶ |

Chaque échantillon cellulaire a été traité de la façon suivante. Après lyophilisation, l'extrait cellulaire est repris par 1 ml d'eau distillée. 100 µl sont soumis au traitement par la phosphatase acide et le contenu en ddA est déterminé. 200 µl seront réservés au dosage direct et sans purification sur cartouche. Les 700 µl restants sont soumis à une chromatographie sur cartouche anionique Sep Pak dans les conditions d'élution précédemment décrites. Des fractions de 500 µl sont collectées. Chaque fraction est diluée 1,6 fois avec de l'eau distillée puis fractionnée en deux. 400 µl seront destinés au dosage direct, 400 µl seront déphosphorylés à l'aide de la phosphatase acide en présence d'acétate de sodium 1 M à 37°C pendant 30 minutes. Les résultats des dosages bruts direct et indirect sont présentés dans la table récapitulative 6.

Après purification sur cartouche anionique, l'immunoréactivité en équivalent ddATP se situe principalement au niveau des fractions 24-31, 32-39 et 42.

L'immunoréactivité, mesurée par dosage direct, localisée dans les fractions 42 n'est pas augmentée par déphosphorylation. Ceci indique la présence de ddATP dans cette fraction. En revanche, l'immunoréactivité présente au niveau des fractions 24-29 et 33-39 se trouve augmentée lors du dosage indirect (après déphosphorylation) indiquant que l'on démasque l'épitope reconnu par l'anticorps. Ces fractions, correspondant à des produits moins retenus sur la cartouche anionique, devraient correspondre respectivement au produit mono et diphosphorylé (ddAMP et ddADP), comme le montre les résultats obtenus chez le patient PRI et illustrés dans le tableau 5 ci-dessous.

**Table 5**

| **Fractions** | **Dosage direct après purification sur cartouche (fmoles/10⁶ cellules)** | **Dosage indirect après purification sur cartouche (fmoles/10⁶ cellules)** |
|---|---|---|
| 24-29 | 20 | 114 |
| 33-39 | 19 | 252 |
| 42 | 91 | 96 |

### VI.3 Bilan des résultats.

Le tableau récapitulatif 6 ci-dessous illustre les résultats des dosages intracellulaires après purification, sur cartouche anionique, des produits issus du métabolisme cellulaire de la ddI.

**Table 6**

| **Patients** | **Nb total de cellules 10⁶** | **ddI plasmatique (M)** | **ddAMP (fmoles/10⁶ cellules)** | **ddADP (fmoles/10⁶ cellules)** | **ddATP (fmoles/10⁶ cellules)** |
|---|---|---|---|---|---|
| | **(8 ml)** | | | | |
| BED | 3,92 | 6,1.10⁻⁶ | 145 | 535 | 6 |
| IPE | 7,84 | 1,95.10⁻⁶ | 24 | 295 | 15 |
| TRI | 6,7 | 3,20.10⁻⁶ | 62 | 174 | 61 |
| PRI | 10,8 | 2,95.10⁻⁶ | 114 | 252 | 91 |

Dans cet exemple, du ddATP a été détecté de manière incontestable chez 4 patients. Chez un patient, il n'a pas été détecté de forme phosphorylée de la ddA, alors que chez 2 des 4 patients, uniquement les formes mono- et diphosphorylées ont été détectées. Il est probable que chez ces patients, soit le ddATP n'est pas encore synthétisé, soit les kinases impliquées dans la troisième étape de phosphorylation sont déficientes. La répétition des dosages à des intervalles réguliers chez les patients permettra de corréler les variations de la concentration intracellulaire en ddATP observées avec les indications sur l'état d'avancement de l'infection au VIH, sur les traitements antérieurs et sur l'échappement thérapeutique éventuel et permettra d'effectuer un meilleur suivi thérapeutique.

Les résultats de la quantification des dérivés phosphorylés de la ddA, obtenus par le dosage brut, direct et indirect ainsi que par le dosage après cartouche sont relativement cohérents entre eux à l'exception d'un seul échantillon.

### VI.4 Conclusions

Les exemples de réalisation de l'invention, avec les dosages effectués sur quatre échantillons et les résultats obtenus, montrent que le dosage immunologique des analogues de nucléosides, objet de la présente invention avec les anticorps également préparés selon une méthode précédemment décrite, peut être utilisé pour la quantification de tous les métabolites phosphorylés de la ddA

L'ensemble des résultats montre que le test immunologique mis au point avec les anticorps est spécifique du ddATP. Cette spécificité est assurée par la spécificité de l'anticorps associée à la purification des molécules à doser lorsqu'elles sont dans les mêmes compartiments (ddI, ddATP, ddADP, et ddAMP) et par le fait qu'elles sont reconnues par l'anticorps avec des affinités différentes.

## Revendications

1. Immunogène, **caractérisé en ce qu'**il comporte un isostère de nucléoside triphosphate dans lequel le groupement phosphate a été remplacé par un groupement citrate couplé à une molécule porteuse.

2. Immunogène selon la revendication 1, **caractérisé en ce que** le nucléoside est choisi parmi le groupe comprenant les nucléosides : Acyclovir, Adénosine, S-Adenosyl-L-methionine, 2',3'-didéoxyadénosine (ddA), 2',3'-didehydro-2',3'-didéoxythymidine (d4T), 2',3'-dideoxy-3'-thiacytidine (3TC), 3'-Azido-3'-deoxythymidine (AZT), Carbovir, Cordycepine, Cytidine, Cytosine-b-D-arabinoside, Deoxycytidine, Deoxytubercidine, 2'-Deoxyuridine, Formycine A, Formycine B, Ganciclovir, Guanosine, Inosine, Puromycine, Ribavirine, Sangivamycine, Thymidine, Tubercidine, Uridine, Abacavir, 3-fluoro-2',3'-dideoxythymidine (FLT), Fura, FdUrd, Thio-Gua, 6-MP, AraC ou Fludarabine phosphate.

3. Immunogène selon la revendication 1, **caractérisé en ce que** le nucléoside est choisi parmi le groupe comprenant les analogues des bases puriques : 6-mercaptopurine, cladribine, fludarabine ou le groupe comprenant les analogues des bases pyrimidiques : 5-fluoro-uracile, cytarabine, gemcitabine, Tenofovir.

4. Immunogène selon l'une quelconque des revendications précédentes, **caractérisé en ce que** la molécule porteuse est choisie parmi une protéine, un polypeptide ou un polysaccharide.

5. Immunogène selon la revendication 4, **caractérisé en ce que** la protéine est choisie parmi le groupe comprenant : l'ovalbumine, le sérum albumine bovine, l'hémocyanine de patelle, la thyroglobuline, des immunoglobulines, la caséine, l'hémoglobine, des toxines bactériennes, de préférence la sous-unité B de la toxine du choléra, la toxine tétanique, la toxine diphtérique, de lectines, de préférence la sous-unité B de la ricine.

6. Utilisation d'un immunogène défini à l'une quelconque des revendications précédentes pour la préparation d'un anticorps ayant une affinité pour un dérivé ddNTP.

7. Procédé d'obtention d'un anticorps ayant une affinité pour un dérivé ddNTP comprenant les étapes suivantes :
a) l'immunisation d'un animal non humain, avec au moins un immunogène défini à l'une quelconque des revendications 1 à 5, lequel immunogène comporte un dérivé citrate de nucléoside couplé à une molécule porteuse,
b) la purification des anticorps polyclonaux produits.

8. Procédé d'obtention d'un anticorps ayant une affinité pour un dérivé ddNTP comprenant les étapes suivantes :
a) l'immunisation de cellules immunocompétentes d'un animal non humain, avec au moins un immunogène défini à l'une quelconque des revendications 1 à 5,
b) l'immortalisation des cellules sécrétant des anticorps ayant une affinité pour ledit dérivé ddNTP au moyen d'une fusion cellulaire des cellules de l'animal immunisé, isolées à partir de la rate ou des ganglions lymphatiques, avec une lignée cellulaire de fusion,
c) l'identification et sélection des clones hybrides immortalisés, hybridomes, sécrétant lesdits anticorps ayant une affinité pour ledit dérivé ddNTP,
d) la purification des anticorps produits par lesdits hybridomes.

9. Procédé selon la revendication 8, **caractérisé en ce que** l'animal immunisé à l'étape (a) du procédé est choisi parmi un mammifère non humain, de préférence un rongeur, tout préférentiellement un rat ou une souris ou une poule.

10. Procédé d'obtention d'un anticorps selon les revendications 8 ou 9, **caractérisé en ce que** les cellules immunocompétentes sont immunisées *in vitro.*

11. Procédé d'obtention d'un anticorps selon les revendications 8 ou 9, **caractérisé en ce que** les cellules immunocompétentes sont immunisées *in vivo.*

12. Procédé selon l'une quelconque des revendication 8 à 11, **caractérisé en ce que** la lignée de fusion, utilisée à l'étape (b) du procédé est choisie parmi une lignée myélomateuse de souris, une lignée myélomateuse de rat ou encore des lignées hétérohybrides.

## Claims

1. Immunogen, **characterised in that** it comprises a triphosphate nucleoside isostere wherein which the phosphate group has been replaced by a citrate grouping paired with a carrier molecule.

2. Immunogen according to claim 1, **characterised in that** the nucleoside is selected from the group comprising the nucleosides: Acyclovir, Adenosine, S-Adenosyl-L-methionine, 2',3'-dideooxyadenosine (ddA), 2',3'-didehydro-2',3'-dideoxythymidine (d4T), 2',3'-dideoxy-3'-thiacytidine (3TC), 3'-Azido-3'-deoxythymidine (AZT), Carbovir, Cordycepine, Cytidine, Cytosine-b-D-arabinoside, Deoxycytidine, Deoxytubercytidine, 2'-Deoxyuridine, Formycine A, Formycine B, Ganciclovir, Guanosine, Inosine, Puromycine, Ribavirine, Sangivamycin, Thymidine, Tubercidine, Uridine, Abacavir, 3-fluoro-2',3'-dideoxythymidine (FLT), Fura, FdUrd, Thio-Gua, 6-MP, AraC or Fludarabine phosphate.

3. Immunogen according to claim 1, **characterised in that** the nucleoside is selected from the group comprising puric bases analogues: 6-mercaptopurine, cladribine, fludarabine or the group comprising pyramidic bases analogues: 5-fluoro-uracil, cytarabine, gemcitabine, Tenofovir.

4. Immunogen according to any of the preceding claims, **characterised in that** the carrier molecule is selected from a protein, a polypeptide or a polysaccharide.

5. Immunogen according to claim 4, **characterised in that** the protein is selected from the group comprising: ovalbumen, bovine albumen serum, patella haemocyanin, thyroglobulin, immunoglobulins, casein, haemoglobin, bacterial toxins, preferably the cholera toxin B sub-unit, tetanic toxin, diphtheric toxin, lectins, preferably the ricin B sub-unit.

6. Use of an immunogen defined in any of the preceding claims for preparing an antibody having an affinity for a ddNTP derivative.

7. Method for obtaining an antibody having an affinity for a ddNTP derivative comprising the following steps:
a) immunisation of a non-human animal, with at least one immunogen defined in any of claims 1 to 5, said immunogen including a nucleoside citrate derivative paired with a carrier molecule,
b) purification of the polyclonal antibodies produced.

8. Method for obtaining an antibody having an affinity for a ddNTP comprising the following steps:
a) immunisation of immunocompetent cells from a non-human animal, with at least one immunogen defined in any of claims 1 to 5,
b) immortalisation of cells secreting antibodies having an affinity for said ddNTP derivative by means of a cellular fusion of the immunised animal cells, isolated from the spleen or lymphatic ganglia, with a cellular fusion line,
c) identification and selection of the immortalised hybrid clones, hybridomes, secreting said antibodies having an affinity for said ddNTP derivative,
d) purification of antibodies produced by said hybridomes.

9. Method according to claim 8, **characterised in that** the animal immunised in step (a) of the method is selected from a non-human mammal, more preferably a rodent, most preferably a rat or a mouse or a chicken.

10. Method for obtaining an antibody according to claim 8 or 9, **characterised in that** the immunocompetent cells are immunised *in vitro.*

11. Method for obtaining an antibody according to claim 8 or 9, **characterised in that** the immunocompetent cells are immunised *in vivo.*

12. Method according to any of claims 8 to 11, **characterised in that** the fusion line, used in step (b) of the method is selected from a mouse myelomatous line, a rat myelomatous line or even heterohybrid lines.

## Patentansprüche

1. Immunstoff, **dadurch gekennzeichnet, dass** er ein Isosteren aus Nukleosidtriphosphat umfasst, worin die Phosphatgruppe durch eine mit einem Trägermolekül gebundene Zitratgruppe ersetzt wurde.

2. Immunstoff nach Anspruch 1, **dadurch gekennzeichnet, dass** das Nukleosid aus der Gruppe der folgenden Nukleoside ausgewählt wurde: Acyclovir, Adenosin, S-Adenosyl-L-Methionin, 2', 3' - Dideoxyadenosin (ddA), 2', 3' - Didehydro-2', 3' Dideoxythymidin (d4T), 2', 3' - Dideoxy-3' -Thiacytidin (3TC), 3' -Azido-3' - Deoxythymidin (AZT), Carbovir, Cordycepin, Cytidin, Cytosin-b-D-Arabinosid, Deoxycytidin, Deoxytubercydin, 2' -Deoxyuridin, Formycin A, Formycin B, Ganciclovir, Guanosin, Inosin, Puromycin, Ribavirin, Sangivamycin, Thymidin, Tubercidin, Uridin, Abacavir, 3-Fluor-2', 3' - Dideoxythymidin (FLT), Fura, FdUrd, Thi-Gua, 6-MP, AraC oder Fludarabinphosphat.

3. Immunstoff nach Anspruch 1, **dadurch gekennzeichnet, dass** das Nukleosid aus der Gruppe der Analoga auf Purin-Basis: 6-Mercaptopurin, Cladribin, Fludarabin oder aus der Gruppe der Analoga auf Pyrimid-Basis: 5-Fluoruracil, Cytarabin, Gemcitabin, Tenofovir gewählt wird.

4. Immunstoff nach einem der vorstehenden Ansprüche, **dadurch gekennzeichnet, dass** als Trägermolekül ein Protein, ein Polypeptid oder ein Polysaccharid gewählt wird.

5. Immunstoff nach Anspruch 4, **dadurch gekennzeichnet, dass** das Protein aus der folgenden Gruppe gewählt wird: Ovalbumin, Bovin Serum Albumin, Patella-Hämocyanin, Thyroglobulin, Immonuglobuline, Casein, Hämoglobin, bakterielle Toxine" vorzugsweise die Untereinheit B des Cholera-Toxins, tetanisches Toxin, diphterisches Toxin, Lectine, vorzugsweise die Untereinheit B von Ricin.

6. Verwendung eines Immunstoffs gemäß der Definition in einem der vorstehenden Ansprüche für die Vorbereitung eines Antikörpers mit einer Affinität für ein Derivat ddNTP.

7. Verfahren zur Erlangung eines Antikörpers mit einer Affinität für ein Derivat ddNTP, bestehend aus den folgenden Phasen:
a) Immunisierung eines nicht menschlichen Lebewesens mit mindestens einem Immunstoff, wie er in einem der Ansprüche 1 bis 5 definiert wird, wobei dieser Immunstoff ein Zitrat-Nukleosidderivat umfasst, das an ein Trägermolekül gebunden ist.
b) Reinigung der erzeugten polyclonen Antikörper.

8. Verfahren zur Erlangung eines Antikörpers mit einer Affinität für ein Derivat ddNTP, bestehend aus den folgenden Phasen:
a) Immunisierung der immunkompetenten Zellen eines nicht menschlichen Lebewesens mit mindestens einem Immunstoff, wie er in einem der Ansprüche 1 bis 5 definiert wird,
b) Immortalisierung der Zellen, die die Antikörper mit einer Affinität für ein Derivat ddNTP ausscheiden, mittels einer Fusion der Zellen des immunisierten Tiers, die über die Milz oder die Lymphknoten mit einer Zellenfusionslinie isoliert wurden,
c) Bestimmung und Auswahl der unsterblich gemachten Hybridclonen, die die Antikörper mit einer Affinität für ein Derivat ddNTP ausscheiden,
d) Reinigung der erzeugten Antikörper durch die erwähnten Hybridome.

9. Verfahren nach Anspruch 8, **dadurch gekennzeichnet, dass** das in der Phase (a) des Verfahrens immunisierte Tier aus der Gruppe der nicht menschlichen Säugetiere gewählt wird, vorzugsweise ein Nagetier, und besonders vorzugsweise eine Ratte bzw. eine Maus oder ein Huhn.

10. Verfahren zur Erlangung eines Antikörpers nach den Ansprüchen 8 oder 9, **dadurch gekennzeichnet, dass** die immunkompetenten Zellen *in vitro* immunisiert werden.

11. Verfahren zur Erlangung eines Antikörpers nach den Ansprüchen 8 oder 9, **dadurch gekennzeichnet, dass** die immunkompetenten Zellen *in vitro* immunisiert werden.

12. Verfahren nach einem der Ansprüche 8 bis 11, **dadurch gekennzeichnet, dass** als in der Phase (b) des Verfahrens verwendete Fusionslinie eine myelomatise Linie der Maus, eine myelomatise Linie der Ratte oder aber heterohybride Linien verwendet werden.
